(19) Europäisches Patentamt / European Patent Office / Office européen des brevets

(11) **EP 3 599 988 B1**

(12) **FASCICULE DE BREVET EUROPEEN**

(45) Date de publication et mention de la délivrance du brevet:
**06.07.2022 Bulletin 2022/27**

(21) Numéro de dépôt: **18717267.1**

(22) Date de dépôt: **29.03.2018**

(51) Classification Internationale des Brevets (IPC):
**A61B 3/11** *(2006.01)*   **A61B 3/06** *(2006.01)*
**A61B 3/14** *(2006.01)*

(52) Classification Coopérative des Brevets (CPC):
**A61B 3/112; A61B 3/066; A61B 3/145**

(86) Numéro de dépôt international:
**PCT/EP2018/058119**

(87) Numéro de publication internationale:
**WO 2018/178257 (04.10.2018 Gazette 2018/40)**

(54) **BIOMARQUEUR DE LA PERCEPTION DES COULEURS CHEZ UN SUJET MAMMIFERE BASE SUR LE MARQUAGE FREQUENTIEL DE LA PUPILLE**

BIOMARKER FÜR DIE FARBWAHRNEHMUNG EINES SÄUGERS BASIEREND AUF DER MARKIERUNG DER PUPILLENFREQUENZ

BIOMARKER OF THE COLOUR PERCEPTION OF A MAMMAL SUBJECT BASED ON PUPIL FREQUENCY TAGGING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.03.2017 FR 1752812**

(43) Date de publication de la demande:
**05.02.2020 Bulletin 2020/06**

(73) Titulaires:
• **Institut du Cerveau et de la Moelle Épinière-ICM**
**75013 Paris (FR)**
• **Centre National de la Recherche Scientifique - CNRS**
**75016 Paris (FR)**
• **Institut National de la Santé et de la Recherche Médicale (INSERM)**
**75013 Paris (FR)**
• **Assistance Publique-Hôpitaux de Paris (APHP)**
**75001 Paris (FR)**
• **Sorbonne Université**
**75006 Paris (FR)**

(72) Inventeurs:
• **POUGET, Pierre**
**75015 Paris (FR)**
• **DAYE, Pierre**
**1420 Braine-L'Alleud (BE)**
• **LORENCEAU, Jean**
**75013 Paris (FR)**

(74) Mandataire: **Nony**
**11 rue Saint-Georges**
**75009 Paris (FR)**

(56) Documents cités:
WO-A1-2015/063598   WO-A1-2015/120438
WO-A1-2016/099402   US-A1- 2009 213 329
US-A1- 2015 245 766

**Description**

DOMAINE DE L'INVENTION

La présente invention concerne l'évaluation de la perception des couleurs chez les mammifères, et plus particulièrement un procédé et un système de génération d'un indicateur ou biomarqueur de la perception des couleurs chez un sujet mammifère, tel qu'un être humain ou un animal.

CONTEXTE DE L'INVENTION

La perception des couleurs est fortement variable d'un sujet mammifère à l'autre.

Cette variabilité s'explique traditionnellement par des concentrations, des répartitions et des sensibilités différentes, entre sujets, des photorécepteurs chromatiques (cônes) dans les zones sollicitées de la rétine, généralement sur et autour de la fovéa.

La perception des couleurs évolue avec l'âge du sujet.

Elle s'altère aussi par un dysfonctionnement lié à une pathologie génétique ou acquise de l'œil (les photorécepteurs chromatiques), des voies optiques ou des aires cérébrales visuelles.

L'évaluation de la perception des couleurs est utile pour de nombreuses applications, médicales et non-médicales.

A titre d'exemple, le document US 2015/245766 utilise la différence de réponse pupillaire d'un patient soumis à des motifs monochromes tels que des flashs blanc (ou rouge) et bleu pour déterminer une détérioration neurologique du patient.

Une méthode d'évaluation, dite d'iso-luminance chromatique, consiste à déterminer lorsqu'une combinaison de couleurs, dont l'une varie dans le temps, est perçue par le sujet comme ayant toutes (généralement deux) la même luminance. Cette évaluation est largement utilisée dans les études psychophysiques et neurophysiologiques du traitement visuel chez les mammifères. Elle permet notamment d'évaluer l'intégrité de la perception des couleurs chez le sujet, mais également d'isoler la contribution relative des cellules sensibles à la luminance dans la perception des couleurs.

Longtemps l'évaluation de l'iso-luminance chromatique est restée subjective car se reposant sur des jugements perceptifs des sujets.

Pour permettre une évaluation objective chez des sujets mammifères non verbaux, tels que les animaux et les bébés, des indicateurs ou biomarqueurs de la perception des couleurs chez un sujet mammifère ont été créés. Ces indicateurs ou biomarqueurs sont en quelque sorte des signatures neurologiques et physiologiques objectives de la perception des couleurs testées.

La publication « Screening for color blindness using optokinetic nystagmus » (Cavanagh P. et al., 1984) décrit par exemple la génération d'un signal représentatif du nystagmus de l'œil du sujet en réponse à un scintillement illusoire dans une grille formée de barres rouges et vertes, l'une des couleurs étant graduellement modifiée dans le temps. Ce signal de réponse est un indicateur de la perception de couleurs chez le sujet testé. Notamment l'iso-luminance entre les deux couleurs testées (rouge et verte) est obtenue au moment où la direction de mouvement de l'œil s'inverse dans le signal du nystagmus.

La publication « A new technique for estimating chromatic isoluminance in humans and monkeys » (Chaudhuri A. et al., 1990) divulgue également la génération d'un signal de réponse opto-cinétique à l'affichage d'un stimulus bicolore dynamique. Un changement de direction du nystagmus est également utilisé pour identifier l'iso-luminance entre les deux couleurs (vert et gris) utilisées.

Ces techniques basées sur la réponse opto-cinétique des sujets comme indicateur de la perception des couleurs requièrent cependant une analyse très précise du mouvement des yeux. En effet, les oscillations involontaires des yeux en réponse au stimulus bicolore dynamique sont généralement brutales et rapides. Des dispositifs d'acquisition d'images sophistiqués (par exemple des caméras 100 images/seconde) et des moyens de traitement performants, notamment pour des traitements temps-réel, sont donc nécessaires.

En outre, pour garantir cette précision, il est nécessaire que le regard du sujet ne bouge pas. Cette contrainte est difficile à tenir pour certains sujets, tels que les animaux et les bébés.

De plus, l'utilisation d'un oculomètre pour la mesure des mouvements oculaires nécessite une procédure de calibration de l'oculomètre. Cette procédure de calibration nécessite aussi une réponse de la part des sujets, rendant ces mesures difficiles sur des sujets non-coopérants.

Il existe ainsi un besoin de disposer d'indicateurs ou biomarqueurs de la perception des couleurs qui soient plus simples à obtenir et moins contraignants.

RESUME DE L'INVENTION

Les inventeurs ont eu l'idée de s'appuyer sur la réponse de la pupille à la variation de luminance. En effet, cette réponse (contraction ou dilatation de la pupille) étant plus lente que le nystagmus, les inventeurs ont pensé que des équipements et des traitements moins sophistiqués seraient alors suffisants.

Dans ce contexte, la présente invention concerne tout d'abord un procédé de génération d'un indicateur ou biomarqueur de la perception des couleurs chez un sujet mammifère, comprenant les étapes suivantes :

soumettre le sujet mammifère à au moins un stimulus multicolore, typiquement bicolore, dynamique comprenant l'affichage, sur un périphérique d'affichage (écran ou tout autre support visuel), d'un motif multicolore, typiquement bicolore, dont au moins deux couleurs sont inversées périodiquement à une fréquence dite de marquage. Le motif multicolore affiche ainsi plusieurs couleurs (dont les deux qui s'inversent) à chaque instant donné,

contrôler une modification dans le temps d'au moins une des deux couleurs du motif multicolore lors de l'affichage du stimulus multicolore dynamique, pour faire varier la luminance affichée de cette couleur (généralement plusieurs fois). La modification de la couleur a pour vocation de faire varier dans le temps la luminance relative entre les deux couleurs (c'est-à-dire une différence de luminance réelle ou perçue),

acquérir, par un dispositif d'acquisition d'images, une réponse oscillatoire d'au moins une pupille du sujet mammifère, lors de l'affichage du stimulus multicolore dynamique, et

générer, à partir de la réponse acquise, un signal représentatif de la puissance de la réponse oscillatoire de la pupille en fonction de la modification dans le temps d'au moins une des deux couleurs (et plus généralement d'une différence de luminance entre les deux couleurs) lors de l'affichage du stimulus multicolore dynamique.

De façon classique, la réponse oscillatoire de la pupille consiste à mesurer l'évolution (constriction et dilatation) du diamètre de la pupille dans le temps. Avantageusement, les pupilles des deux yeux peuvent être analysées séparément ou en combinaison (par exemple au travers d'une moyenne).

Du fait de la réponse oscillatoire lente de la pupille, la fréquence de marquage utilisée est assez faible et, de plus, éloignée des autres fréquences physiologiques du sujet.

Par cet éloignement des fréquences physiologiques, le rapport signal/bruit de la réponse oscillatoire de la pupille se trouve être naturellement élevé. Il permet alors d'obtenir un signal représentatif de la puissance de la réponse oscillatoire qui représente fidèlement la réponse pupillaire, sans contamination. Ce signal représentatif de la réponse rétinienne et/ou perception (propre au sujet) relative des couleurs testées peut donc servir directement ou être lié directement à un indicateur ou biomarqueur fiable de la discrimination et/ou perception des couleurs.

Le signal généré peut alors être utilisé dans des applications médicales ou non-médicales, comme évoqué par la suite, et par exemple pour la détermination d'une iso-luminance chromatique entre deux couleurs testées.

En outre, par l'usage d'une fréquence de marquage faible, généralement de l'ordre de 0,1 Hz à 5 Hz, par exemple de 1,3 ou 1,4 Hz, des dispositifs d'acquisition vidéo conventionnels, typiquement des caméras 25 images par seconde (comme celles équipant les ordinateurs et téléphones mobiles), sont suffisants. Les traitements, notamment temps réel, des signaux acquis s'en trouvent alors substantiellement allégés.

Corrélativement, l'invention concerne un système de génération d'un indicateur ou biomarqueur de la perception des couleurs chez un sujet mammifère, comprenant :

un périphérique d'affichage,

un système informatique de stimulation du sujet mammifère par stimulus multicolore, typiquement bicolore, dynamique, le système informatique commandant l'affichage, sur le périphérique d'affichage, d'un motif multicolore, typiquement bicolore, dont au moins deux couleurs sont inversées périodiquement à une fréquence dite de marquage,

un contrôleur de couleurs configuré pour modifier dans le temps au moins une des deux couleurs du motif multicolore lors de l'affichage du stimulus multicolore dynamique, pour faire varier la luminance affichée de cette couleur,

un dispositif d'acquisition d'images pour acquérir une réponse oscillatoire d'au moins une pupille du sujet mammifère, lors de l'affichage du stimulus multicolore dynamique, et

un générateur d'indicateur ou biomarqueur configuré pour générer, à partir de la réponse acquise, un signal représentatif de la puissance de la réponse oscillatoire de la pupille en fonction de la modification dans le temps d'au moins une des deux couleurs lors de l'affichage du stimulus multicolore dynamique.

Ce système présente des avantages similaires à ceux du procédé décrit ci-dessus.

Des caractéristiques optionnelles du procédé selon l'invention sont par ailleurs définies dans les revendications dépendantes. Le système selon l'invention peut également comprendre des moyens configurés pour mettre en œuvre ces caractéristiques optionnelles.

Dans un mode de réalisation, le procédé comprend en outre une étape consistant à déterminer la configuration bicolore d'iso-luminance des deux couleurs (c'est-à-dire les valeurs de ces deux couleurs) du stimulus multicolore dynamique correspondant à un minimum du signal représentatif de la puissance de la réponse oscillatoire de la pupille. Les inventeurs ont constaté qu'à raison de l'inversion périodique des deux couleurs dans le motif, la pupille oscille à la fréquence correspondante (de marquage) avec une réponse d'autant plus puissante que le différentiel perçu d'intensité des deux couleurs est important. Aussi, la détermination du minimum du signal représentatif de la puissance permet de déterminer l'iso-luminance chromatique perçue par le sujet pour les deux couleurs testées, c'est-à-dire lorsque la différence de luminance que l'on fait varier dans le temps entre les deux couleurs testées est minimale selon la perception du sujet. La correspondance d'iso-luminance chromatique peut être utilisée comme indicateur ou marqueur de la perception de la couleur chez un sujet. En effet, elle peut être comparée à celle obtenue pour d'autres sujets.

Les calculs mis en œuvre pour la détermination de l'iso-luminance chromatique sont excessivement simplifiés par rapport aux techniques connues. En outre, la position du minimum dans le signal représentatif de la puissance est indépendante de l'unité utilisée pour mesurer la réponse oscillatoire de la pupille. Aussi, cette configuration permet de s'affranchir d'une calibration (étalonnage) du dispositif d'acquisition d'images.

Dans un mode de réalisation, l'autre couleur parmi les deux couleurs du motif multicolore est maintenue fixe pendant l'affichage du stimulus multicolore dynamique.

Dans un autre mode de réalisation, la fréquence de marquage est fonction du sujet. Notamment, le procédé peut comprendre en outre une étape préalable de détermination de la fréquence de marquage, comprenant la soumission du sujet à au moins un flash lumineux de calibration, la mesure d'un temps de réponse moyen de la pupille du sujet mammifère au flash lumineux de calibration, et la fixation de la fréquence de marquage en fonction du temps de réponse moyen mesuré.

Ces dispositions permettent notamment, à faible coût temporel, d'améliorer la fiabilité des mesures effectuées, par exemple la configuration d'iso-luminance chromatique pour les couleurs testées. En outre, elles permettent d'ajuster au mieux la durée du test (soumission au stimulus multicolore, typiquement bicolore, dynamique), et notamment de la réduire.

En variante ou en combinaison, le motif multicolore utilisé est fonction du sujet, par exemple fonction de la réponse oscillatoire de la pupille aux flashs lumineux. Cela permet notamment de tenir compte d'une éventuelle pathologie du sujet qui altère la sensibilité spatiale des yeux du sujet. A titre d'exemple, le motif multicolore peut être positionné dans une zone préférentielle de l'écran compte tenu du sujet, par exemple le haut ou le bas de l'écran, ou encore un quart particulier de l'écran.

Dans un mode de réalisation, le contrôle de la modification dans le temps de la couleur comprend une modification graduelle par incréments (ou paliers). Cela permet d'acquérir des réponses oscillatoires stables, notamment sur une fenêtre glissante d'analyse. En particulier, une fréquence de modification de la couleur (par incrément) est inférieure à la fréquence de marquage. Préférentiellement, la fréquence de modification de la couleur est un sous-multiple de la fréquence de marquage. Cela permet d'avoir plusieurs alternances des mêmes deux couleurs dans le motif multicolore, typiquement bicolore, affiché, et donc d'acquérir une réponse oscillatoire de meilleure qualité.

Dans un mode de réalisation, le sujet mammifère est soumis à deux stimuli multicolores, typiquement bicolores, dynamiques successifs basés sur deux couples de couleurs différents. Cette disposition permet d'obtenir un indicateur relatif complet de la perception des couleurs chez le sujet. En effet, l'espace colorimétrique est généralement tri-dimensionnel (par exemple RGB, pour Red-Green-Blue, soit Rouge-Vert-Bleu) de telle sorte que la connaissance de la perception relative de deux couples de couleurs permet de déduire (par de simples calculs par exemple) la perception relative de l'ensemble des couples de couleurs. Cette perception relative est par exemple la correspondance d'iso-luminance chromatique.

Dans un mode de réalisation, l'affichage de couleurs sur le périphérique d'affichage est contrôlé à l'aide de triplets de valeurs Rouge-Vert-Bleu, et les deux couleurs du motif multicolore sont des couleurs pures pour lesquels deux des trois composantes Rouge, Vert, Bleu sont nulles. Ainsi, la modification dans le temps de la couleur se fait simplement par l'incrément progressif de la troisième composante non nulle.

Dans ce cas, les deux couples de couleurs indiqués ci-dessus sont choisis, dans l'espace RGB, parmi les couples RG, RB et GB, où R, G, B sont respectivement les couleurs rouge pure, verte pure et bleue pure.

Le signal représentatif de la puissance peut être formé de différentes manières selon le traitement appliqué au signal de réponse acquis.

Dans un premier mode de réalisation, le signal est un signal représentatif de la puissance d'oscillation de la pupille à la fréquence de marquage et/ou à une ou plusieurs de ses harmoniques, représentatif de l'évolution de la composante fréquentielle, à ladite fréquence de marquage et/ou à une ou plusieurs de ses harmoniques, de la réponse oscillatoire de la pupille. On entend, par « harmoniques », les multiples et/ou sous-multiples de la fréquence de marquage. Typiquement, on peut s'intéresser, outre la fréquence de marquage $F_{tag}$, à la demi-harmonique $F_{tag}/2$ et/ou à la double harmonique $2*F_{tag}$.

Le passage dans le domaine fréquentiel améliore la résistance de l'indicateur ou biomarqueur alors généré, au bruit. Par exemple, la génération du signal représentatif de la puissance d'oscillation de la pupille à la fréquence de marquage comporte l'application, à la réponse acquise, d'une transformée de Fourier rapide discrète sur une fenêtre temporelle glissante et la mémorisation, pour chaque fenêtre temporelle, de la valeur de la composante fréquentielle à la fréquence de marquage et/ou à une ou plusieurs de ses harmoniques du spectre fréquentiel obtenu. Ces traitements peuvent être avantageusement réalisés en temps réel, avec peu de ressources mémoires.

Notamment, la largeur de la fenêtre temporelle glissante est choisie au moins égale à la période associée à la fréquence de marquage, par exemple au moins le double de cette période.

Selon une autre caractéristique particulière, la fenêtre temporelle glissante est décalée d'un échantillon de la réponse acquise, à chaque nouvelle application de la transformée de Fourier rapide discrète. La précision temporelle des échantillons dépend généralement du dispositif d'acquisition d'images utilisé et de sa configuration. Avantageusement, la présente invention permet d'utiliser une fréquence d'échantillonnage de 25 Hz, correspondant à la définition d'une caméra classique (25 images par secondes). Ainsi, grâce à la disposition ci-dessus, le signal représentatif de la puissance d'oscillation généré a la meilleure définition possible compte tenu du dispositif d'acquisition utilisé.

Dans un mode de réalisation particulier, la génération du signal représentatif de la puissance d'oscillation de la pupille à la fréquence de marquage et/ou à une ou plusieurs de ses harmoniques comporte en outre l'approximation d'un signal formé des valeurs mémorisées de la composante fréquentielle à la fréquence de marquage et/ou à une ou plusieurs de ses harmoniques par au moins une fonction mathématique, par exemple une fonction par morceaux qui peut combiner une ou plusieurs sous-fonctions parmi une fonction affine, une fonction exponentielle. Des techniques classiques d'approximation et de sélection de chaque meilleure sous-fonction pour une partie du signal formé des valeurs mémorisées peuvent être utilisées. Le recours à une telle approximation permet d'obtenir un indicateur ou biomarqueur relativement simple, et donc de simplifier les traitements ultérieurs, par exemple l'usage de cet indicateur pour personnaliser des opérations informatiques ou pour évaluer l'évolution d'une pathologie ou l'efficacité d'un traitement contre une telle pathologie.

Dans un second mode de réalisation, la génération du signal représentatif de la puissance comporte la détermination d'une amplitude de variation du diamètre de la pupille en réponse à chaque inversion des couleurs du motif multicolore, typiquement bicolore, ledit signal représentatif de la puissance étant formé à partir des amplitudes ainsi déterminées. Ces amplitudes, c'est-à-dire les différences de diamètre de la pupille à chaque réponse pupillaire, sont en effet représentatives de la puissance de la réponse pupillaire, à un coefficient près (le temps de réponse de la pupille).

Toute autre méthode permettant d'obtenir un signal représentatif de la puissance de la réponse oscillaire (c'est-à-dire la réponse à chaque changement contrôlé dans l'affichage du motif multicolore) de la pupille peut être utilisée.

Pour l'ensemble de ces méthodes l'analyse peut être effectuée sans filtrage préalable particulier. Par ailleurs et avantageusement, la présente invention permet d'utiliser une fréquence d'échantillonnage de 25 Hz, correspondant à la définition d'une caméra classique (25 images par secondes). Ainsi, le signal représentatif de la puissance d'oscillation est généré avec la meilleure définition possible compte tenu du dispositif d'acquisition utilisé.

Dans un mode de réalisation, le procédé peut en outre comprendre la détermination du minimum du signal représentatif de la puissance ainsi généré, par exemple le minimum de la fonction par morceaux construite ci-dessus ou le minimum du signal des amplitudes d'oscillation de la pupille. Ce minimum permet alors d'identifier la configuration multicolore, typiquement bicolore d'iso-luminance perçue (ou les configurations multicolores d'iso-luminance si par exemple le motif inclut plusieurs couples de couleurs dont la luminance de l'une d'entre elles est modifiée dans le temps).

Dans un mode de réalisation, le procédé comprend en outre une étape de filtrage de la réponse oscillatoire acquise, l'étape de filtrage comprenant l'interpolation, préférentiellement linéaire, d'un signal de réponse oscillatoire lors d'un clignement d'œil. Cela permet de corriger des artéfacts ponctuels dans la courbe.

Nombreuses pathologies entraînent une dégradation de la perception des couleurs chez le mammifère. Il est un besoin de disposer d'indicateurs ou biomarqueurs efficaces de ces pathologies. Dans ce contexte, l'invention concerne également un procédé de détermination d'un indicateur ou biomarqueur caractéristique d'une pathologie chez un sujet mammifère, comprenant les étapes suivantes :

générer au moins deux indicateurs ou biomarqueurs de la perception des couleurs chez le sujet mammifère à deux instants distincts respectifs, à l'aide de la méthode décrite ci-dessus en utilisant le même stimulus multicolore dynamique (c'est-à-dire par exemple les deux mêmes couleurs qui s'inversent, la même fréquence de marquage, etc.), et

déterminer un indicateur de modification entre les deux signaux représentatifs de puissance ainsi générés.

En effet, les inventeurs ont constaté que les dégradations de perception des couleurs liées à ces pathologies altèrent (et donc modifient) directement les signaux représentatifs de la puissance de la réponse oscillatoire de la pupille lors des deux moments de test. Ils ont alors eu l'idée d'établir un indicateur caractéristique de ces pathologies à partir de l'évolution, dans le temps, de ces signaux représentatif de la puissance d'oscillation pupillaire.

Cet indicateur de modification, c'est-à-dire l'indicateur ou biomarqueur caractéristique d'une pathologie, peut

**EP 3 599 988 B1**

ainsi être utilisé dans un processus plus spécifique de détection d'une pathologie, de suivi d'une pathologie, de suivi de l'efficacité d'un traitement contre une pathologie, etc.

Par ailleurs, les deux yeux du sujet concerné ne perçoivent généralement pas les couleurs de la même manière. La différence de perception peut être légère ou significative. Il peut donc être prévu d'obtenir un tel indicateur de modification pour chacun des deux yeux. En effet, certains types de pathologies (certaines maladies neurologiques par exemple) peuvent être détectés lorsque les deux indicateurs sont significatifs, alors que d'autres types de pathologies, à effet plus local (maladies ophtalmologiques telles le glaucome ou une maladie rétinienne, mais également certaines maladies neurologiques telles la sclérose en plaque), peuvent être détectés lorsqu'un seul des indicateurs (celui de l'œil affecté) est significativement dégradé. Notamment pour des maladies neurologiques telles la sclérose en plaque, l'indicateur ainsi généré permet d'identifier quel œil se trouve être affecté par la maladie.

Corrélativement, l'invention concerne un système de détermination d'un indicateur ou biomarqueur caractéristique d'une pathologie chez un sujet mammifère, comprenant :

un système de génération d'un indicateur ou biomarqueur de la perception des couleurs chez un sujet mammifère, tel que défini plus haut, et
un module de traitement configuré pour déterminer un indicateur de modification entre deux signaux représentatif de puissance générés, par le système de génération, à deux instants distincts pour le même sujet en utilisant le même stimulus multicolore dynamique.

Des caractéristiques optionnelles du procédé de détermination d'un indicateur ou biomarqueur selon l'invention sont par ailleurs définies dans les revendications dépendantes. Le système selon l'invention peut également comprendre des moyens configurés pour mettre en œuvre ces caractéristiques optionnelles.

Dans un mode de réalisation, l'indicateur de modification inclut une différence entre les valeurs moyennes des deux signaux représentatif de puissance ainsi générés.

Préférentiellement, l'étape de génération d'au moins deux indicateurs ou biomarqueurs comprend la détermination, pour chacun des deux instants, de la configuration bicolore d'iso-luminance (c'est-à-dire les valeurs des deux couleurs inversées) du stimulus multicolore dynamique correspondant à un minimum du signal représentatif de la puissance ainsi généré, et l'indicateur de modification comprend une différence entre les valeurs de couleurs des deux configurations bicolores déterminées.

Un tel indicateur ou biomarqueur est simple à obtenir, sans effort excessif pour le sujet suivi. Il permet de détecter une altération de la perception des couleurs, et donc une dégradation de l'œil (les photorécepteurs chromatiques), des voies optiques (nerf optique) ou des aires cérébrales visuelles.

Ainsi, cet indicateur ou biomarqueur permet de diagnostiquer, mais également de suivre l'évolution, des pathologies telles que, de façon non exhaustive, la sclérose en plaques, les hépatites (le court-circuitage du foie pouvant s'accompagner d'une dégradation neurologique due à des intoxications), le diabète (pouvant également s'accompagner d'une dégradation neurologique), les maladies neurodégénératives (Alzheimer, Parkinson), neuro-développementales (schizophrénie, autisme), neuro-vasculaires (AVC), les intoxications (affectant le système neurologique), la dégénérescence maculaire liée à l'âge, le glaucome et les maladies rétiniennes, etc.

Dans ce contexte, l'invention concerne également l'utilisation de l'indicateur de modification obtenu pour un sujet mammifère par le procédé ci-dessus de détermination d'un indicateur ou biomarqueur caractéristique d'une pathologie chez un sujet mammifère, dans le diagnostic d'une pathologie ou le suivi de l'évolution d'une pathologie chez le sujet.

Cette utilisation peut notamment comprendre la comparaison de l'indicateur de modification avec une valeur seuil. A titre d'exemple, si la différence d'iso-luminance chromatique entre deux mesures effectuées (les deux configurations bicolores minimisant le signal représentatif de la puissance d'oscillation) est grande (supérieure à la valeur seuil), cela peut signifier que le système neurologique du sujet s'est dégradé, et qu'ainsi un traitement curatif en cours d'essai n'est pas efficace, ou à l'inverse que le système neurologique du sujet s'est amélioré (si la différence montre un retour vers une configuration d'iso-luminance plus conforme). Bien entendu, la valeur seuil peut être ajustée en fonction de l'écart entre les deux instants de mesure, mais également en fonction de la pathologie suivi et du sujet concerné.

BREVE PRESENTATION DES FIGURES

D'autres particularités et avantages de l'invention apparaîtront encore dans la description ci-après, illustrée par les dessins ci-joints, dans lesquels :

- la **Figure 1** illustre un système de génération d'un indicateur ou biomarqueur de la perception des couleurs chez un sujet mammifère selon un mode de réalisation de l'invention ;
- la **Figure 2** illustre schématiquement un stimulus bicolore dynamique selon un mode de réalisation de l'invention ;

- la **Figure 3a** illustre un motif ou profil de modification d'une des couleurs du motif bicolore du stimulus de la **Figure 2** selon un mode de réalisation ;
- la **Figure 3b** représente un exemple d'une réponse oscillatoire d'une pupille en réponse à un stimulus bicolore dynamique du type celui de la **Figure 2** selon un mode de réalisation ;
- la **Figure 3c** illustre schématiquement un spectre fréquentiel issu de l'application d'une transformée de Fourier rapide discrète sur une fenêtre d'analyse du signal de la **Figure 3b** selon un mode de réalisation ;
- les **Figures 3d** et **3d'** illustrent des signaux représentatifs de la puissance d'oscillation de la pupille, et une fonction par morceaux les approximant, selon différents modes de réalisation ;
- la **Figure 3e** illustre la détermination d'une valeur d'iso-luminance chromatique sur le profil de modification de la **Figure 3a** selon un mode de réalisation ;
- la **Figure 4** illustre, par un organigramme, un procédé de génération d'un indicateur ou biomarqueur de la perception des couleurs chez un sujet mammifère selon des modes de réalisation de l'invention ; et
- la **Figure 5** illustre, par un organigramme, un procédé de détermination d'un indicateur ou biomarqueur caractéristique d'une pathologie chez un sujet mammifère selon des modes de réalisation de l'invention.

DESCRIPTION DETAILLEE DE MODES DE REALISATION DE L'INVENTION

La présente invention s'intéresse à la perception ou discrimination des couleurs chez les mammifères pour en faire un biomarqueur caractéristique de chaque sujet testé. Elle tire profit de la réponse oscillatoire pupillaire lente (entre 0,1 Hz à 5 Hz) d'un sujet mammifère à un stimulus lumineux oscillatoire pour générer une signature neurologique objective de la perception relative des couleurs par un sujet.

Une méthode par marquage fréquentiel de la pupille (« pupil frequency-tagging method » en langue anglo-saxonne) est utilisée à une fréquence de marquage $F_{tag}$ adaptée à la vitesse de réponse pupillaire, et qui avantageusement est éloignée des autres fréquences physiologiques associées aux mouvements de réponse de l'œil du sujet. A titre d'exemple $F_{tag}$ est de l'ordre de 1,3 à 1,4 Hz voire moins.

Cette méthode est robuste aux bruits résultant d'autres mouvements oculaires du sujet et ne nécessite pas de calibration de la part du sujet.

Cette méthode permet par exemple de déterminer l'iso-luminance chromatique perçue par un sujet à partir de la signature neurologique obtenue.

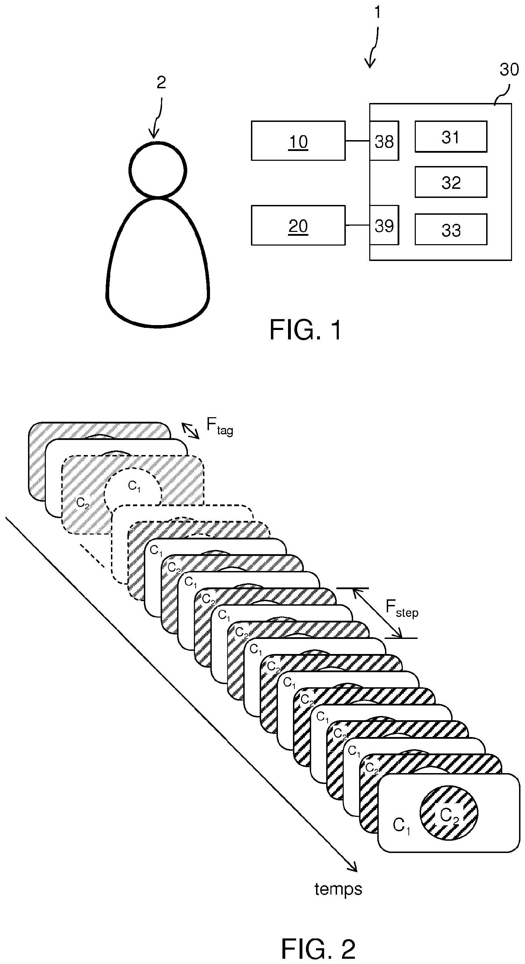

La **Figure 1** illustre un système 1 de génération d'un indicateur ou biomarqueur de la perception des couleurs chez un sujet mammifère 2.

Il comporte un écran d'affichage 10, un dispositif d'acquisition d'images 20 et un système informatique de commande et de traitement 30.

L'écran d'affichage 10 est une dalle de pixels, par exemple de résolution 1920x1080 pixels avec une fréquence d'affichage de par exemple 60 Hz, pilotées par une carte vidéo du système 30. Chaque pixel est formé de trois composantes couleurs sur 8 bits chacune (c'est-à-dire pouvant prendre des valeurs de 0 à 255) : Rouge, Vert, Bleu (RGB). Bien entendu d'autres définitions de pixels (nombre de bits par couleur, espace colorimétrique) peuvent être envisagées dans le cadre de la présente invention.

L'écran 10 peut être positionné centré face aux yeux du sujet, dans un plan perpendiculaire au regard, et à une distance fixe du sujet 2. Pour éviter tout mouvement du sujet 2, la tête de celui-ci peut être stabilisée en la faisant reposer par appui du menton et appui frontal sur des supports appropriés.

Le dispositif d'acquisition d'images 20 est typiquement une caméra sensible à l'infrarouge ou tout type de capteur permettant d'enregistrer un diamètre pupillaire, également disposée face au sujet afin de pouvoir acquérir des images d'une ou plusieurs pupilles du sujet 2.

Pour des raisons de simplification des explications, on se limitera par la suite à l'acquisition d'images d'une seule pupille du sujet 2. Bien entendu, des traitements similaires peuvent être réalisées sur l'acquisition d'images des deux pupilles, par exemple effectuer des moyennes ou corroborer les résultats obtenus pour une pupille par les résultats de l'autre pupille, ou enfin analyser l'altération d'une pupille relativement à l'autre.

Compte tenu de la fréquence de marquage $F_{tag}$ relativement faible, l'usage de caméras 20 sur étagère, du type 25 images/seconde, est possible. Notamment les caméras embarquées sur les dispositifs électroniques conventionnels (téléphones mobiles, ordinateurs, tablettes numériques, caméra portatives) peuvent être utilisées pour autant qu'elles soient sensibles aux infrarouges.

Bien entendu, des caméras disposant de fréquences d'acquisition supérieures à 25 im/s peuvent également être utilisées.

Lorsqu'une caméra opère sur un spectre fréquentiel plus large que celui de l'infrarouge, un filtrage (physique ou électronique) peut être prévu selon des techniques connues (et non décrites ici) afin d'obtenir en fin d'acquisition uniquement des images dans le spectre infrarouge ou à tout le moins permettre de déterminer le diamètre de la pupille observée.

Le système informatique de commande et de traitement 30 comporte un module informatique de stimulation du sujet mammifère par stimulus multicolore, typiquement bicolore, dynamique 31, un contrôleur de couleurs 32 et un générateur d'indicateur ou biomarqueur 33. Ces différents éléments sont mis en œuvre par logiciel car il s'agit essentiellement de traitements visant à commander un affichage sur l'écran 10 ou à traiter des données acquises par le dispositif 20. A cette fin, le système 30 comporte également une carte vidéo classique 38 à laquelle est relié l'écran 10, et une carte d'acquisition vidéo classique 39 à laquelle est relié le dispositif d'acquisition 20.

Le système 30 peut en outre comprendre des moyens d'entrées/sorties (clavier, souris, carte réseau) pour permettre à un opérateur de paramétrer le système et de déclencher les processus et applications de l'invention.

Le module informatique de stimulation du sujet mammifère par stimulus multicolore, typiquement bicolore, dynamique 31 commande l'affichage, sur l'écran 10, d'un motif multicolore, typiquement bicolore, dont au moins deux couleurs sont inversées périodiquement à la fréquence de marquage $F_{tag}$.

Le motif multicolore en question affiche plusieurs couleurs à chaque instant donné. Au moins deux de ces couleurs sont alors inversées l'une l'autre périodiquement.

La **Figure 2** illustre schématiquement un exemple de motif bicolore formé des couleurs $C_1$ et $C_2$, un fond d'écran homogène d'une des couleurs (ici laissé en blanc pour une meilleure lisibilité) avec, en son centre, un cercle homogène de l'autre couleur (ici en hachuré pour une meilleure lisibilité). Le stimulus bicolore dynamique consiste à créer une alternance entre les deux couleurs $C_1$ et $C_2$ à la fréquence $F_{tag}$. Le motif bicolore peut être présenté sur un fond blanc par exemple.

Préférentiellement, les deux couleurs $C_1$ et $C_2$ du motif bicolore sont des couleurs pures, c'est-à-dire des couleurs pour lesquels deux des trois composantes Rouge, Vert, Bleu sont nulles. Dans l'espace colorimétrique RGB, $C_1$ et $C_2$ sont ainsi soit RG, soit RB, soit GB, où R, G, B sont respectivement les couleurs rouge pure $(r_i,0,0)$, verte pure $(0,g_i,0)$ et bleue pure $(0,0,b_i)$.

A noter que 'C_1 et C_2 peuvent être choisies comme étant la même couleur pure (RR, GG, BB), notamment pour des tests de contrôle. Bien entendu, d'autres choix de couples de couleurs $(C_1, C_2)$ n'impliquant pas nécessairement des couleurs pures peuvent être envisagés.

Le contrôleur de couleurs 32 est configuré pour modifier dans le temps au moins une des deux couleurs du motif multicolore, typiquement bicolore **(Figure 2)** lors de l'affichage du stimulus multicolore, typiquement bicolore dynamique sur l'écran 10, pour faire varier la luminance affichée de cette couleur (généralement plusieurs fois). Pour des couleurs pures, la variation de luminance est facile à obtenir, consistant simplement à modifier la seule composante ($r_i$ ou $g_i$ ou $b_i$) non nulle.

Cette modification d'une des deux couleurs a pour objectif de faire varier dans le temps la luminance relative entre les deux couleurs considérées, c'est-à-dire faire varier une différence de luminance entre les deux couleurs, différence réelle ou perçue par le sujet.

Dans un mode de réalisation, l'autre couleur du motif, disons $C_1$, est maintenue fixe pendant l'affichage du stimulus bicolore dynamique. Cela signifie que pendant tout le test (l'ensemble des images affichées de la **Figure 2),** la couleur $C_1$ est affichée avec le même triplet RGB. Bien entendu, cette autre couleur peut également être modifiée dans le temps (par exemple en sens contraire de la première couleur). Ce qui importe ici est de déterminer le décalage entre les deux couleurs qui assure une iso-luminance perçue. En première approximation, ce décalage peut ensuite être appliqué à n'importe quelle valeur d'une des couleurs, pour obtenir la valeur de l'autre couleur en configuration d'iso-luminance.

La modification dans le temps de la couleur $C_2$ est préférentiellement réalisée par paliers croissants dans une plage de valeurs possibles (par exemple de 0 à 255 pour la seule composante modifiée) ou dans une plage de valeurs à tester, ou par paliers décroissants (par exemple de 255 à 0) pendant le test. Pour des couleurs non pures, la luminance résultante (différentes formules à partir des composantes RGB sont à la disposition de l'homme de l'art) sera choisie croissante ou décroissante pendant le test.

En variante, une modification de la couleur $C_2$ par dichotomie autour d'une première valeur d'iso-luminance déterminée lors d'un premier test (basé selon les techniques de la présente invention ou selon d'autres méthodes) peut être envisagée.

D'autres exemples de motifs multicolores peuvent être utilisés, par exemple incluant un plus grand nombre de couleurs ou un motif multicolore affichant deux ou plus couples différents de couleurs qui sont inversées deux à deux comme expliqué par exemple ci-dessus. Egalement, des motifs spatialement différents peuvent être envisagés dans la zone d'affichage sur l'écran 10, par exemple un affichage sur l'un ou l'autre des quarts d'écran ou bien encore un affichage sur la partie haute (ou droite) ou basse (ou gauche) de l'écran.

Pour simplifier les explications qui suivent, il est fait référence principalement à un motif bicolore dont les deux couleurs affichées simultanément sont inversées (lequel peut par exemple être affiché sur un fond uniforme, résultant en définitive en un affichage multicolore).

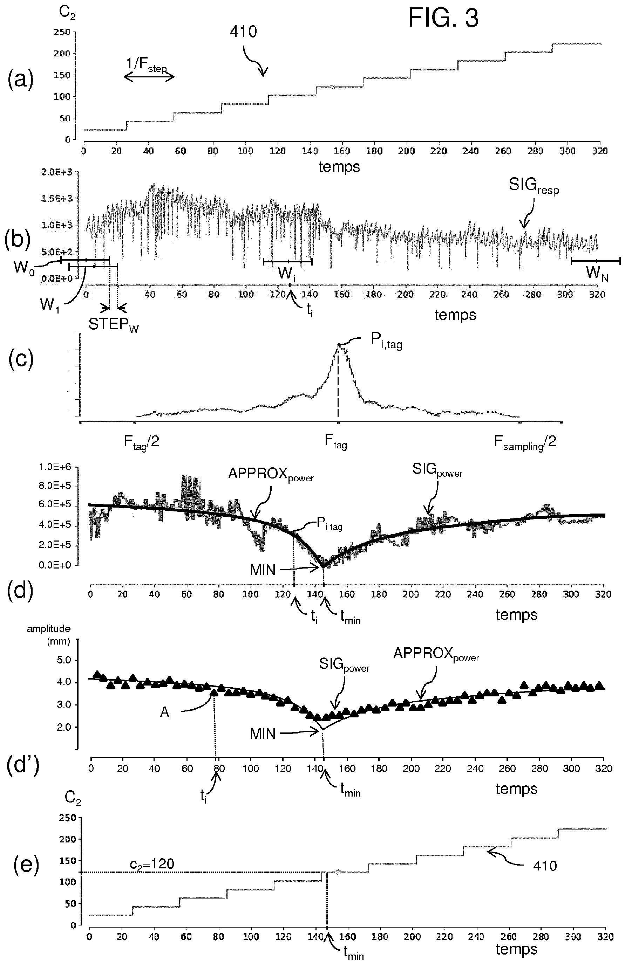

La **Figure 3a** illustre un exemple de contrôle de la modification dans le temps de la couleur $C_2$ consistant en une modification graduelle par incréments (ou escaliers). La différence de luminance entre les deux couleurs varie ainsi dans le temps. Dans cet exemple, on fait varier la composante non nulle de la couleur $C_2$ entre 20 et 220 (les extrêmes ne sont pas significatifs), par paliers de 20 unités. Bien entendu, d'autres plages testées, avec d'autres paliers peuvent être utilisés pour réduire ou allonger le temps de test, mais également réduire ou améliorer la précision de la détermination par exemple de l'iso-luminance chromatique.

Notamment, une précision plus importante (plage plus étroite et paliers plus petits) peut être utilisée, dans un second test, autour d'une valeur approximative d'iso-luminance (c'est-à-dire la valeur de $C_2$ ayant la même luminance perçue par le sujet 2 que la valeur fixe de $C_1$) déterminée au préalable lors d'un premier test (basé selon les techniques de la présente invention ou selon d'autres méthodes).

Comme illustré sur la **Figure 2,** la fréquence $F_{step}$ d'incrément de la valeur de $C_2$ est choisie inférieure à la fréquence de marquage $F_{tag}$, par exemple 3 fois inférieure **(Figure 2** - signifiant que trois images affichées consécutives ont la même valeur de couleur $C_2$), 10 fois inférieure environ **(Figure 3a)** voire plus, ou éventuellement un sous-multiple de $F_{tag}$.

De retour à la **Figure 1,** le générateur d'indicateur ou biomarqueur 33 est configuré pour générer, à partir d'une réponse oscillatoire $SIG_{resp}$ d'au moins une pupille du sujet 2 acquise par le dispositif d'acquisition 20, un signal dit de puissance d'oscillation $SIG_{power}$ de la pupille. Un exemple de réponse oscillatoire est illustré en **Figure 3b,** alors que des exemples de signaux de puissance d'oscillation de la pupille sont illustrés en **Figures 3d** et **3d'.**

Ce signal $SIG_{power}$ est représentatif de la puissance de la réponse oscillatoire de la pupille en fonction de la modification dans le temps **(Figure 3a)** d'au moins une des couleurs lors de l'affichage du stimulus bicolore dynamique.

Différents modes de réalisation peuvent être envisagés pour obtenir ce signal $SIG_{power}$.

Une première méthode rapide consiste à déterminer une amplitude $A_i$ de variation du diamètre de la pupille en réponse à chaque inversion (à $t_i$) des couleurs du motif bicolore. Chaque amplitude $A_i$ quantifie la modification du diamètre de la pupille lors de la réponse pupillaire. Une telle amplitude est représentative de la puissance de la réponse pupillaire, à un coefficient près (le temps de réponse de la pupille). Le signal $SIG_{power}$ est alors formé des amplitudes ainsi déterminées (voir **Figure 3d'** dans laquelle les triangles représentent schématiquement chaque amplitude $A_i(t_i)$ déterminée).

Une deuxième méthode plus résistante au bruit consiste à travailler dans le domaine fréquentiel de la réponse oscillatoire $SIG_{resp}$. Dans ce cas, le signal $SIG_{power}$ est préférentiellement un signal représentatif de la puissance d'oscillation de la pupille à la fréquence de marquage, représentatif de l'évolution de la composante fréquentielle $P_{i,tag}$, à ladite fréquence de marquage $F_{tag}$, de la réponse oscillatoire $SIG_{resp}$ de la pupille en fonction de la modification dans le temps **(Figure 3a)** de la couleur $C_2$ lors de l'affichage du stimulus bicolore dynamique **(Figure 2).** Le signal $SIG_{power}$ est alors formé des composantes fréquentielles $P_{i,tag}$ ainsi déterminées (voir **Figure 3d).**

Il est à noter que la détermination de ces composantes fréquentielles ne requiert pas nécessairement l'acquisition des réponses oscillatoires complètes à chaque inversion des couleurs. En effet, des techniques connues permettent d'obtenir ces composants fréquentielles à partir d'un cycle partiel de la réponse oscillatoire de la pupille. Dans un mode de réalisation préféré, on s'appuie sur des réponses oscillatoires complètes.

En variante ou en combinaison avec la première harmonique $F_{tag}$, il est possible de s'intéresser aux harmoniques de la fréquence de marquage $F_{tag}$, notamment les sous-harmoniques telles que la demi-harmonique $F_{tag}/2$ et/ou les harmoniques multiples telles que la double ou deuxième harmonique $2*F_{tag}$. Dans ce cas, chaque harmonique est traitée séparément comme décrit par la suite, leurs résultats pouvant être combinés (au travers d'une moyenne ou pour ajuster le résultat de la première harmonique, etc.) pour obtenir notamment une unique configuration bicolore d'iso-luminance.

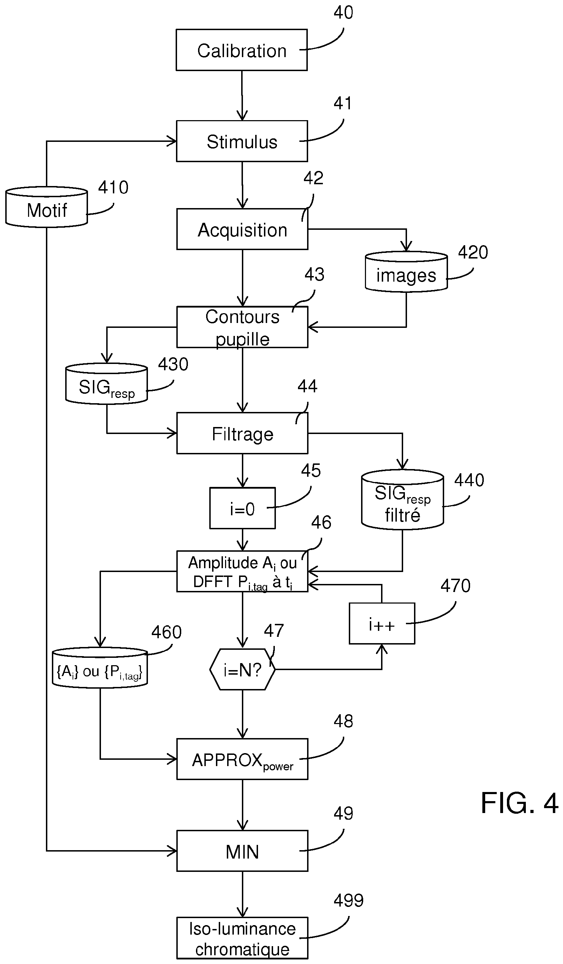

Ainsi, à partir du signal $SIG_{power}$ de n'importe quelle harmonique traitée, le générateur d'indicateur ou biomarqueur 33 peut notamment déterminer l'iso-luminance de $C_2$ par rapport à $C_1$ (fixe). Comme expliqué par la suite en référence à la **Figure 4** en lien avec les **Figures 3d** et **3e,** cette iso-luminance chromatique correspond à la configuration bicolore d'iso-luminance du stimulus bicolore dynamique correspondant à un minimum du signal $SIG_{power}$. En effet, l'amplitude de la réponse pupillaire et donc sa puissance d'oscillation suivent de près les variations de luminance du stimulus affiché. Elles sont donc minimales lorsque les variations de luminance sont minimales, c'est-à-dire lors de l'iso-luminance chromatique perçue par le sujet du motif bicolore affiché.

En référence maintenant à la **Figure 4,** on décrit un exemple de procédé de génération d'un indicateur ou biomarqueur de la perception des couleurs chez un sujet mammifère selon les enseignements de l'invention.

Au démarrage du système 1, une étape optionnelle 40 permet de calibrer le système 1 au sujet 2 testé. En particulier cette étape a pour but de déterminer la fréquence $F_{tag}$ en fonction du sujet 2 et/ou le motif à utiliser. En effet, le temps de réponse pupillaire varie, parfois fortement, d'un sujet à l'autre. Aussi, pour réduire la durée du test ou garantir des mesures pertinentes, il est préférable que $F_{tag}$ soit aussi grande que possible tout en évitant qu'elle ne dépasse la vitesse de réponse pupillaire du sujet.

Par ailleurs, un motif différencié spatialement par rapport au point de fixation du sujet peut être envisagé afin d'isoler des atteintes affectant spécifiquement certaines zones de la rétine du sujet. Par exemple, une différence de perception de couleur haut-bas peut exister pour des patients atteints d'Alzheimer, alors que des zones particulières sont difficilement perçues par des patients atteints localement de DMLA (ces zones correspondants aux parties dégénérées dans la rétine compte tenu de la pathologie).

L'étape 40 de calibration consiste de façon générale à enregistrer la réponse pupillaire du sujet (généralement en constriction), pour en déduire un temps de réponse pupillaire et ainsi une fréquence $F_{tag}$ à utiliser (par exemple à l'aide d'une table de correspondance), et/ou une zone de meilleure sensibilité du sujet définissant une zone préférentielle d'affichage du motif bicolore.

A titre d'exemple, le sujet 2 peut être soumis à au moins un flash lumineux de calibration commandé par le système 30 et affiché sur l'écran 10 (par exemple un écran blanc soudain à partir d'un écran sombre ou une zone blanche soudaine dans l'écran sombre, l'emplacement de cette zone blanche pouvant varier lors du test de calibration).

Une acquisition, par le dispositif 20, des images de la ou des réponses pupillaires du sujet 2 (au flash ou aux flashes multiples) permet d'obtenir un signal représentatif de la variation (constriction) de la pupille (notamment de son diamètre), à partir duquel le temps de réponse (éventuellement moyen sur plusieurs réponses) de la pupille peut être mesuré par des techniques classiques (par exemple le temps mis, à compter du flash, pour atteindre 90% de la constriction en réponse). Classiquement, le temps de réponse pupillaire d'un individu est de l'ordre de 0,5 secondes à 2 secondes.

A partir de ce temps de réponse mesuré ou calculé, l'étape 40 fixe la fréquence $F_{tag}$. Ce peut être fait à l'aide d'une table de correspondances (qui associe, à des plages de temps de réponse, des valeurs de $F_{tag}$ respectives) afin d'avoir un nombre limité de valeurs de fréquences de marquage. En variance, la période associée à la fréquence de marquage $F_{tag}$ peut être mise au double du temps de réponse ou à un autre multiple du temps de réponse mesuré.

Par défaut, une valeur $F_{tag}$ d'environ 1,3 à 1,4 Hz peut être utilisée.

Egalement à partir de réponses pupillaires à l'affichage d'un flash en plusieurs zones de l'écran, alors que le sujet conserve un point de fixation, le dispositif détermine une zone de meilleure sensibilité du sujet. Cette zone peut alors être utilisée pour l'affichage du motif bicolore sur une partie seulement de l'écran. Aussi, des motifs spatialement différenciés sont finalement affichés en fonction des sujets concernés.

A l'étape 41, le sujet 2 est soumis à au moins un stimulus multicolore, typiquement bicolore, dynamique.

Bien que la description qui suit s'intéresse à la soumission du sujet à un seul stimulus bicolore, il peut être prévu de le soumettre successivement à deux stimuli bicolores dynamiques successifs basés par exemple sur deux couples de couleurs différents, afin notamment d'obtenir une perception relative des couleurs du sujet sur l'ensemble de l'espace colorimétrique tridimensionnel RGB. A titre d'exemple, un test déterminant l'iso-luminance chromatique du couple RG pour le sujet peut être combiné à un deuxième test ultérieur déterminant l'iso-luminance chromatique du couple GB. La perception relative entre les couleurs Rouge et Bleu peut alors se déduire directement, de sorte que la perception relative de toutes les couleurs dans l'espace colorimétrique est connue pour le sujet.

Egalement, bien que le stimulus utilisé ici soit bicolore au sens où seulement deux couleurs sont interverties, l'une d'entre elles au moins variant dans le temps, il peut être prévu que le stimulus multicolore utilisé comprennent plusieurs couples différents de couleurs interverties à la fréquence $F_{tag}$, affichés simultanément sur le même écran.

Lors de l'étape 41, le module informatique de stimulation du sujet mammifère par stimulus bicolore dynamique 31 commande l'affichage du motif bicolore éventuellement dans une zone préférentielle d'affichage et l'inversion périodique des deux couleurs $C_1$ et $C_2$ à la fréquence $F_{tag}$ **(Figure 2)**. Dans le même temps, la luminance de la couleur $C_2$ est modifiée dans le temps selon un profil de modification 410, sous le contrôle du contrôleur de couleurs 32. Le profil de modification peut être incrémental comme montré sur la **Figure 3a** pour la composante non nulle de $C_2$.

A l'étape 42, le dispositif d'acquisition d'images 20 filme et acquiert des images, dans le spectre infrarouge, de la pupille surveillée du sujet 2. Ces images sont transmises au système 30 et stockées en mémoire 420. L'acquisition est réalisée à une fréquence d'échantillonnage $F_{sampling}$ de 25 à 1000 images par seconde, selon les capacités du dispositif 20.

A l'étape 43, les images acquises sont traitées à l'aide d'un algorithme de détection de contours de la pupille et de mesure du diamètre de la pupille. Ces mesures permettent de dresser un signal de réponse oscillatoire $SIG_{resp}$ de la pupille lors de l'affichage du stimulus bicolore dynamique, représentant l'évolution du diamètre de la pupille dans le temps.

La **Figure 3b** illustre un exemple de réponse oscillatoire $SIG_{resp}$ de la pupille à un stimulus bicolore dynamique Rouge-Vert, dont la couleur rouge pure est fixée à (140,0,0) et la couleur verte pure suit la courbe de la **Figure 3a** ($F_{step}$=0,0345 soit des paliers d'environ 29 secondes). La fréquence $F_{tag}$ est fixée à 0,345 Hz.

La courbe $SIG_{resp}$ ainsi obtenue est stockée en mémoire 430 du système 30.

A noter que les traitements de détection de contours et de génération de $SIG_{resp}$ peuvent être embarqués dans le dispositif d'acquisition 20 afin de réduire le volume de données à transmettre au système informatique 30.

La réponse oscillatoire $SIG_{resp}$ peut être exploitée telle quelle aux étapes 45 à 49 décrites ci-dessous. En effet, l'utilisation de la fréquence $F_{tag}$, éloignée des fréquences de bruits physiologiques, garantit une bonne robustesse de cette réponse.

Il peut cependant être prévu, de façon optionnelle, un filtrage de cette réponse aux fins notamment de supprimer des artéfacts dans le signal.

Dans ce cas, le procédé se poursuit à l'étape optionnelle 44 de filtrage de la réponse oscillatoire $SIG_{resp}$ acquise.

Ce filtrage a notamment pour objectif de supprimer du bruit dans le signal, par exemple le bruit résultant d'un clignement de l'œil où aucune pupille n'a pu être détectée par l'algorithme de détection des contours).

Dans un mode de réalisation, l'étape 44 consiste à interpoler, préférentiellement de façon linéaire, le signal de réponse oscillatoire $SIG_{resp}$ lors d'un ou plusieurs clignements d'œil, c'est-à-dire lorsque l'algorithme de détection de contours n'a pas pu détecter de pupille dans les images. Des techniques classiques d'interpolation peuvent être utilisées.

A titre d'exemple, un filtrage basé sur la méthode Savitzky-Golay peut être mis en œuvre.

Le signal ainsi filtré est conservé en mémoire 440 du système 30.

C'est à partir de ce signal $SIG_{resp}$ filtré ou non que le signal $SIG_{power}$ est généré.

Dans le premier mode de réalisation évoqué ci-dessus, le générateur 33 peut déterminer les instants $t_i$ d'inversion des couleurs dans le stimulus bicolore affiché, puis déterminer dans chaque réponse oscillatoire correspondante, l'amplitude $A_i(t_i)$ de la variation du diamètre pupillaire du sujet 2.

Il peut s'agit simplement de déterminer la différence entre la valeur du diamètre au moment de l'inversion de couleur et la valeur extrémale opposée (maximale ou minimale) du diamètre dans une fenêtre temporelle correspondant sensiblement au temps de réponse du sujet.

Bien entendu, d'autres méthodes d'évaluation de l'amplitude peuvent être envisagées, comme par exemple utiliser un pourcentage de la valeur extrémale du diamètre ou la valeur du diamètre à 90% (ou moins) de la réponse.

L'ensemble des amplitudes calculées, associées aux instants respectifs d'inversion des couleurs, forment le signal $SIG_{power}$ **(Figure 3d')** utilisé aux étapes 48 et 49 ci-dessous.

Dans le deuxième mode de réalisation basé sur le domaine fréquentiel, le signal $SIG_{resp}$ obtenu (éventuellement filtré) est transformé dans le domaine spectral pour analyser l'évolution de la composante fréquentielle $F_{tag}$ et/ou de ses harmoniques. Pour ce faire, on applique au signal $SIG_{resp}$ une transformée de Fourier rapide discrète (DFFT) sur une fenêtre temporelle glissante $W_i$. On obtient ainsi un spectre fréquentiel correspondant à la portion de signal analysée par la fenêtre. Puis, on mémorise, pour chaque fenêtre temporelle d'analyse $W_i$, la valeur $P_{i,tag}$ de la composante fréquentielle à la fréquence $F_{tag}$ (et/ou d'une ou plusieurs harmoniques).

La suite de la description s'intéresse à la composante fréquentielle $F_{tag}$. Une approche similaire peut être utilisée pour traiter n'importe quelle harmonique d'intérêt.

La **Figure 3b** illustre les fenêtres temporelles d'analyse $W_i$ utilisées depuis la première fenêtre $W_0$ jusqu'à la dernière fenêtre $W_N$. Pour assurer la présence de la composante fréquentielle à $F_{tag}$ dans le spectre, la largeur de la fenêtre temporelle glissante $W_i$ est au moins égale à la période $T_{tag}$ associée à la fréquence de marquage $F_{tag}$. Dans l'exemple de la figure, on choisit par exemple une largeur égale à $2 * T_{Tag}$ (ou supérieure). Ainsi, dès la fenêtre $W_0$, centrée sur $t_0$, une composante $P_{0,tag}$ non nulle peut être obtenue.

Chaque fenêtre d'analyse $W_i$ est centrée sur (donc associée à) l'instant $t_i$. Préférentiellement, on associe une fenêtre $W_i$ à chaque instant d'échantillonnage, afin d'obtenir la résolution maximale du signal représentatif de la puissance oscillatoire formé des valeurs $\{P_{i,tag}\}$. Dans ce cas, la fenêtre temporelle glissante $W$ est décalée d'un échantillon de la réponse $SIG_{resp}$ acquise, à chaque nouvelle application de la transformée de Fourier rapide. Bien entendu, le pas $STEP_W$ de décalage de la fenêtre d'analyse $W$ peut être supérieur à une seule période d'échantillonnage ($T_{sampling}=1/F_{sampling}$) utilisée dans cet exemple. Un pas $STEP_W$ fixé à plusieurs périodes d'échantillonnage permet de réduire les calculs et la taille mémoire requise.

Pour obtenir un signal représentatif de la puissance d'oscillation $SIG_{power}$ de la pupille à la fréquence $F_{tag}$ représentatif de l'évolution de la composante fréquentielle $F_{tag}$ de la réponse oscillatoire $SIG_{resp}$ de la pupille, le procédé peut tout d'abord comprendre l'initialisation d'une variable 'i' à zéro (pour traiter chaque échantillon) à l'étape 45.

Puis, la DFFT est appliquée à la portion du signal $SIG_{resp}$ définie par la fenêtre temporelle courante $W_i$ centrée sur l'échantillon 'i' à traiter (c'est-à-dire $t_i$). La

**Figure 3c** illustre un exemple schématique de spectre fréquentiel obtenu. On notera l'absence de composantes fréquentielles en deçà de la fréquence correspondant à la largeur de la fenêtre $W_i$ et au-delà de la demi-fréquence d'échantillonnage (critère de Nyquist). Des composantes d'harmoniques de $F_{tag}$ peuvent donc être présentes.

Ce graphique montre que la composante fréquentielle $P_{i,tag}$ à $F_{tag}$ présente un excellent rapport signal/bruit, du fait que $F_{tag}$ est éloignée des fréquences physiologiques du sujet 2 éventuellement parasitaires. La valeur $P_{i,tag}$ est obtenue et mémorisée en mémoire 460. Il s'agit de l'étape 46.

Il est entendu que la DFFT, puisqu'elle opère sur des valeurs discrètes (échantillons), ne produit pas nécessairement de composante fréquentielle exactement à la fréquence $F_{tag}$. Aussi, la valeur $P_{i,tag}$ peut être celle de la composante fréquentielle obtenue la plus proche de $F_{tag}$, ou une approximation (linéaire par exemple) entre les deux (ou plus) composantes fréquentielles entourant $F_{tag}$, ou encore une moyenne de deux ou plus composantes fréquentielles entourant $F_{tag}$.

A noter que l'utilisation d'une fréquence d'échantillonnage $F_{sampling}$ et/ou d'une taille de fenêtre $W$ aussi grandes que possible accroît le nombre d'échantillons fréquentiels dans le spectre, notamment autour de $F_{tag}$.

Après l'étape 46, on détermine si toutes les DFFT ont été réalisées (test 47 vérifiant que i=N, nombre d'échantillons à traiter sur la période de test), sinon on incrémente la variable 'i' (étape 470) pour réaliser la DFFT du signal sur

la fenêtre $W_i$ suivante.

Lorsque la DFFT a été appliquée à l'aide de toutes les fenêtres (c'est-à-dire sur tous les morceaux de la réponse $SIG_{resp}$), la mémoire 460 stocke l'ensemble des valeurs $P_{i,tag}$ qui forment ensemble le signal $SIG_{power}$ de la pupille à la fréquence $F_{tag}$ (voir **Figure 3d).**

Le signal $SIG_{power}$ généré selon l'une quelconque des méthodes constitue déjà un indicateur ou biomarqueur de la perception des couleurs chez le sujet 2. Il s'agit en effet d'une signature neurologique robuste du sujet 2. Comme on le verra par la suite, l'analyse de son évolution dans le temps peut permettre de détecter une pathologie ou une aggravation de la pathologie. C'est le cas par exemple de la sclérose en plaques.

Des étapes optionnelles 48 et 49 permettent de raffiner cet indicateur ou biomarqueur. La forme générale du signal $SIG_{power}$ montre une zone centrale plus basse que les zones latérales.

A l'étape optionnelle 48, un algorithme d'approximation du signal $SIG_{power}$ est mis en place afin notamment de faire correspondre ce signal à une fonction mathématique. Notamment, on cherche à faire correspondre le signal à une fonction par morceaux, où chaque morceau peut correspondre à une fonction affine, une fonction exponentielle ou toute autre fonction classique.

Dans l'exemple de la **Figure 3d** on utilise une fonction exponentielle par morceaux (deux morceaux) $APPROX_{power}$ afin de correspondre à la partie globalement décroissante du signal (à gauche) et à la partie globalement croissante du signal (à droite). Des techniques classiques d'approximation par mise en correspondance peuvent être utilisées, sans avoir à les détailler ici.

Le signal $APPROX_{power}$ ainsi obtenu peut également être utilisé comme indicateur ou biomarqueur de la perception des couleurs chez le sujet 2.

L'étape également optionnelle 49 consiste à déterminer la valeur $c_2$ de la couleur $C_2$ qui minimise le signal de puissance $SIG_{power}$ ou son approximation $APPROX_{power}$. Cela passe par la détermination du minimum MIN du signal $SIG_{power}$ ou $APPROX_{power}$, selon des techniques classiques et la détermination de la valeur de $C_2$ à l'instant $t_{min}$ correspondant à ce minimum de signal, à l'aide du profil de modification 410 **(Figure 3a** reprise en **Figure 3e).**

Dans l'exemple présent, la valeur $c_2(t_{min})=120$ est obtenue pour $C_2$. En d'autres termes, le sujet 2 perçoit une iso-luminance chromatique relative entre les couleurs pures rouge et verte pour les valeurs $C_1=(140,0,0)$ et $C_2=(0,120,0)$ pour l'exemple montré.

La configuration d'iso-luminance 499 ainsi obtenue peut servir d'indicateur ou biomarqueur de la perception des couleurs chez le sujet 2.

Cette configuration peut être combinée avec une ou plusieurs autres configurations correspondantes d'iso-luminance déterminées à partir d'une ou plusieurs harmoniques de $F_{tag}$.

Il est à noter que dans le cas où le stimulus multicolore dynamique utilisé comporte plusieurs couples différents de couleurs que l'on inverse à la fréquence $F_{tag}$, le minimum MIN du signal $SIG_{power}$ ou $APPROX_{power}$ correspond à une configuration d'iso-luminance globale des différents couples de couleurs, sans que cette configuration ne corresponde spécifiquement à une configuration d'iso-luminance de chaque couple de couleurs pris isolément.

Dans un mode de réalisation, la configuration d'iso-luminance obtenue peut être confirmée en reproduisant le test avec un profil de modification 410 inverse (décroissant par paliers de 220 à 20 par exemple). Notamment, une moyenne des deux valeurs $c_2$ obtenues lors de ces deux tests peut être calculée pour représenter la configuration moyenne d'iso-luminance du sujet 2 pour les couleurs $C_1$ et $C_2$.

La méthode ci-dessus a été testée sur 4 sujets humains pendant 14 sessions de 45 minutes et 7 sujets mammifères non-humains pendant une session de 20 minutes. La fréquence de marquage retenue a été 0,345 Hz, adaptée à l'ensemble des sujets étudiés. Le motif bicolore utilisé a été celui de la **Figure 2,** avec une couleur fixe et l'autre variant par escalier (comme illustré schématiquement sur la **Figure** 3). L'écran plat 10, de définition 1920x1080 pixels, 8 bits par couleur, 60 Hz a été fixé à une distance de 67 cm des sujets.

Les inventeurs ont bien constaté, pour l'ensemble des sujets, une réduction maximale de la puissance des oscillations pupillaires induites par les variations de luminance du stimulus à la configuration d'iso-luminance du motif bicolore testé.

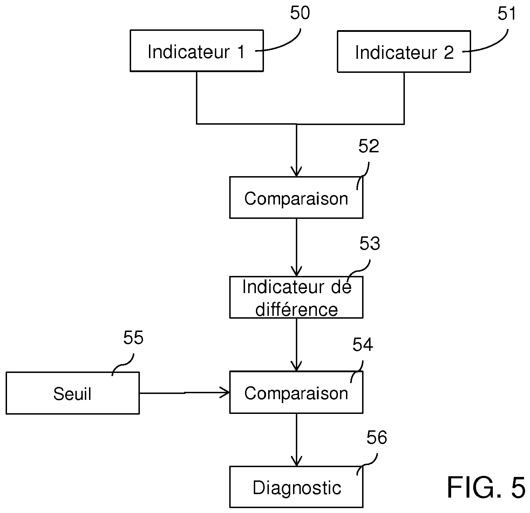

Comme on va le voir maintenant en référence à la **Figure 5,** ces différents indicateurs ou biomarqueurs peuvent être utilisés à des fins médicales, notamment dans la détermination d'un indicateur ou biomarqueur caractéristique d'une pathologie chez un sujet mammifère, et pouvant donc intervenir dans un processus de diagnostic.

Un système de détermination d'un indicateur ou biomarqueur caractéristique d'une pathologie chez un sujet mammifère peut être similaire au système 1 de la **Figure 1** dans lequel un module de traitement 34 (non représenté) est prévu et configuré pour déterminer un indicateur de modification entre deux signaux représentatifs de la puissance d'oscillation de la pupille générés, par le système de génération, à deux instants distincts pour le même sujet en utilisant le même stimulus bicolore dynamique.

La **Figure 5** illustre un exemple de procédé de détermination d'un indicateur ou biomarqueur caractéristique d'une pathologie chez un sujet mammifère, et son utilisation dans un processus de diagnostic.

Le procédé comprend la génération d'au moins deux indicateurs ou biomarqueurs de la perception des couleurs chez le sujet à deux instants distincts respectifs $t_1$ et $t_2$. De préférence, on utilise les enseignements de l'invention et notamment la méthode décrite ci-dessus en lien avec les **Figures 1** à **4**.

Les deux indicateurs ou biomarqueurs sont obtenus, aux étapes 50 et 51, en utilisant le même stimulus multicolore, typiquement bicolore, dynamique : même couleur fixe $C_1$, même profil de modification 410 de la couleur $C_2$, la même fréquence de marquage $F_{tag}$, etc.

Les deux indicateurs ou biomarqueurs sont de même nature et sont par exemple deux signaux $SIG_{power}(t_1)$ et $SIG_{power}(t_2)$ 460, deux signaux d'approximation $APPROX_{power}(t_1)$ et $APPROX_{power}(t_2)$, ou deux configurations d'iso-luminance 499 (ou plus simplement les deux valeurs non nulles $c_2(t_t)$ et $c_2(t_2)$ de $C_2$ correspondant à l'iso-luminance chromatique des deux tests effectués).

Ces deux indicateurs ou biomarqueurs sont ensuite comparés à l'étape 52 pour déterminer un indicateur 53 représentatif d'une modification entre les deux signaux représentatifs de la puissance d'oscillation de la pupille.

Par exemple, l'indicateur de modification 53 est la différence entre les valeurs moyennes de ces deux signaux :

$$\text{moyenne}[SIG_{power}(t_1)] - \text{moyenne}[SIG_{power}(t_2)],$$

ou

$$\text{moyenne}[APPROX_{power}(t_1)] - \text{moyenne}[APPROX_{power}(t_2)].$$

En variante, l'indicateur de modification 53 est la différence entre les valeurs de couleurs des deux configurations d'iso-luminance chromatique: $c_2(t_1) - c_2(t_2)$.

C'est cet indicateur de modification 53 qui constitue un indicateur ou biomarqueur efficace caractéristique d'une éventuelle pathologie chez le sujet.

L'indicateur de modification 53 peut alors être utilisé dans le diagnostic d'une pathologie ou le suivi de l'évolution d'une pathologie chez le sujet.

Par exemple à l'étape 54, l'indicateur de modification 53 est comparé à une valeur seuil 55. Si l'indicateur de modification 53 dépasse cette valeur seuil, alors une pathologie est diagnostiquée ou une dégradation de la pathologie est prononcée (étape 56).

Il a ainsi été constaté qu'une altération de l'œil (les photorécepteurs chromatiques), des voies optiques (nerf optique) ou des aires cérébrales visuelles conduit à une diminution, parfois nette, de la puissance oscillatoire de la pupille et/ou à une modification nette de la perception des couleurs.

Ainsi, à titre d'exemple, l'indicateur de modification 35 peut être utilisé pour suivre une éventuelle intoxication d'un patient opéré d'une hépatite par court-circuitage du foie. Notamment, si la valeur verte d'iso-luminance chromatique avec le rouge pur (140,0,0) chute de plus de 40 unités par rapport à la valeur relevée avant opération, alors une suspicion d'intoxication est diagnostiquée.

Il a notamment été constaté que cet indicateur ou biomarqueur 53 est efficace pour la détection des phases progressives de la sclérose en plaques (SEP) ou le suivi de cette progression en cas de traitement médical (parfois expérimental). Or les techniques actuelles pour cette détection sont notamment basées sur les potentiels évoqués visuels (PEV) qui sont sensibles à des artéfacts de mouvement du sujet, qui nécessitent de longues sessions d'enregistrements et qui requièrent des équipements complexes (électrodes). L'utilisation de l'indicateur ou biomarqueur 53 selon la présente invention s'affranchit de ces contraintes.

Il a également été constaté que cet indicateur ou biomarqueur 53 est approprié au suivi de l'évolution neurologique de certaines pathologies ou résultant d'interventions dues à ces pathologies, par exemple le diabète, l'hépatite (à raison des intoxications post-opératoires). Il permet donc également de détecter toute pathologie altérant la perception des couleurs (par altération du système neurologique du sujet), telle que les maladies neurodégénératives (Alzheimer, Parkinson), neuro-développementales (schizophrénie, autisme), neuro-vasculaires (AVC), les intoxications (affectant le système neurologique), la dégénérescence maculaire liée à l'âge, le glaucome et les maladies rétiniennes, etc.

En effet, l'altération de zones neurologiques (nerfs optiques, aires cérébrales visuelles) conduits à une modification, parfois nette, de la perception des couleurs par le sujet. A l'opposé, un sujet sain conserve une perception des couleurs relativement constante dans le temps (même si elle se modifie légèrement avec le temps).

La publication « Colour vision deficiencies in Alzheimer's disease » (Patche M. et al., 2003) a par exemple montré l'altération de la vision des couleurs chez les sujets atteints par la maladie d'Alzheimer. La publication « Abnormalities in color vision and contrast sensitivity in Parkinson's disease » (Price M.J. et al., 1992) en a fait de même pour la maladie de Parkinson. La publication « Reversible colour vision defects in obstructive jaundice» (Varnek L. et al., 1981) fait également le lien entre un altération de la perception des couleurs et des intoxications liées à des traitements hépatiques. La publication « Diabetic retinopathy seeing beyond glucose-induced microvascular disease » (Antonetti D.A. et al., 2006) lie diabète et altération de la rétine, et donc de la perception des couleurs.

Certaines pathologies (par exemple des maladies neurologiques) affectent les deux yeux du sujet étudié. Aussi, un indicateur/biomarqueur 53 peut être généré pour chacun des deux yeux. Dans ce cas, il est déterminé si chaque indicateur est significativement dégradé. En variante, une moyenne des deux marqueurs peut être utilisée.

D'autres pathologies ont des effets plus localisés, affectant généralement un seul œil. C'est le cas notamment de maladies ophtalmologiques (glaucome, maladie rétinienne), mais également le cas de certaines maladies neurologiques (par exemple la sclérose en plaque peut affecter un seul nerf optique et provoquer une névrite optique locale). De telles pathologies peuvent être détectées lorsqu'un seul des deux indicateurs/biomarqueurs 53 générés est significativement dégradé. De façon réciproque, ces indicateurs 53 permettent de localiser la zone d'effet (c'est-à-dire l'œil) d'une maladie (par exemple dans le cas de la sclérose en plaque évoquée ci-dessus).

**Revendications**

1. Procédé de génération d'un indicateur ou biomarqueur ($SIG_{power}$, $APPROX_{power}$, 499) de la perception des couleurs chez un sujet mammifère (2), comprenant les étapes suivantes :

   soumettre (41) le sujet mammifère à au moins un stimulus multicolore, typiquement bicolore, dynamique comprenant l'affichage, sur un périphérique d'affichage (10), d'un motif multicolore, typiquement bicolore, dont au moins deux couleurs sont inversées périodiquement à une fréquence dite de marquage ($F_{tag}$),
   contrôler (41) une modification dans le temps d'au moins une ($C_2$) des deux couleurs du motif multicolore lors de l'affichage du stimulus multicolore dynamique, pour faire varier la luminance affichée de cette couleur,
   acquérir (42, 43, 44), par un dispositif d'acquisition d'images (20), une réponse oscillatoire ($SIG_{resp}$) d'au moins une pupille du sujet mammifère, lors de l'affichage du stimulus multicolore dynamique, et
   générer (46, 48), à partir de la réponse acquise, un signal ($SIG_{power}$, $APPROX_{power}$) représentatif de la puissance de la réponse oscillatoire ($SIG_{resp}$) de la pupille en fonction de la modification dans le temps d'au moins une des deux couleurs lors de l'affichage du stimulus multicolore dynamique.

2. Procédé selon la revendication 1, comprenant en outre une étape consistant à déterminer la configuration bicolore d'iso-luminance des deux couleurs du stimulus multicolore dynamique correspondant à un minimum (MIN) du signal généré ($SIG_{power}$, $APPROX_{power}$).

3. Procédé selon la revendication 1 ou 2, dans lequel l'autre couleur ($C_1$) du motif multicolore est maintenue fixe pendant l'affichage du stimulus multicolore dynamique.

4. Procédé selon l'une des revendications 1 à 3, dans lequel la fréquence de marquage ($F_{tag}$) est fonction du sujet (2).

5. Procédé selon la revendication 4, comprenant en outre une étape préalable (40) de détermination de la fréquence de marquage ($F_{tag}$), comprenant la soumission du sujet à au moins un flash lumineux de calibration, la mesure d'un temps de réponse moyen de la pupille du sujet mammifère au flash lumineux de calibration, et la fixation de la fréquence de marquage en fonction du temps de réponse moyen mesuré.

6. Procédé selon l'une des revendications 1 à 5, dans lequel le sujet mammifère (2) est soumis à deux stimuli multicolores, typiquement bicolores, dynamiques successifs basés sur deux couples de couleurs différents.

7. Procédé selon l'une des revendications 1 à 6, dans lequel le signal généré est un signal représentatif de la puissance d'oscillation de la pupille à la fréquence de marquage et/ou à une ou plusieurs de ses harmoniques, représentatif de l'évolution de la composante fréquentielle, à ladite fréquence de marquage et/ou à une ou plusieurs de ses harmoniques, de la réponse oscillatoire de la pupille.

8. Procédé selon la revendication 7, dans lequel la génération (46, 48) du signal représentatif de la puissance d'oscillation ($SIG_{power}$, $APPROX_{power}$) de la pupille à la fréquence de marquage ($F_{tag}$) et/ou à une ou plusieurs de ses harmoniques comporte l'application (46), à la réponse acquise ($SIG_{resp}$), d'une transformée de Fourier rapide discrète sur une fenêtre temporelle glissante ($W_i$) et la mémorisation, pour chaque fenêtre temporelle, de la valeur ($P_{i,tag}$) de la composante fréquentielle à la fréquence de marquage et/ou à une ou plusieurs de ses harmoniques du spectre fréquentiel obtenu.

9. Procédé selon l'une des revendications 1 à 6, dans lequel la génération (46, 48) du signal comporte la détermination d'une amplitude de variation du diamètre de la pupille en réponse à chaque inversion des couleurs du motif multicolore, ledit signal ($SIG_{power}$, $APPROX_{power}$) étant formé à partir des amplitudes ainsi déterminées.

10. Système (1) de génération d'un indicateur ou biomarqueur ($SIG_{power}$, $APPROX_{power}$, 499) de la perception des couleurs chez un sujet mammifère (2), comprenant :

   un périphérique d'affichage (10),
   un système informatique (31) de stimulation du sujet mammifère par stimulus multicolore, typiquement bicolore, dynamique, le système informatique commandant l'affichage, sur le périphérique d'affichage, d'un motif multicolore, typiquement bicolore, dont au moins deux couleurs sont inversées périodiquement à une fréquence dite de marquage ($F_{tag}$),
   un contrôleur de couleurs (32) configuré pour modifier dans le temps au moins une ($C_2$) des deux couleurs du motif multicolore lors de l'affichage du stimulus multicolore dynamique, pour faire varier la luminance affichée de cette couleur,
   un dispositif d'acquisition d'images (20) pour acquérir une réponse oscillatoire ($SIG_{resp}$) d'au moins une pupille du sujet mammifère, lors de l'affichage du stimulus multicolore dynamique, et
   un générateur d'indicateur ou biomarqueur (33) configuré pour générer, à partir de la réponse acquise, un signal ($SIG_{power}$, $APPROX_{power}$) représentatif de la puissance de la réponse oscillatoire ($SIG_{resp}$) de la pupille en fonction de la modification dans le temps d'au moins une des deux couleurs lors de l'affichage du stimulus multicolore dynamique.

11. Système de détermination d'un indicateur ou biomarqueur (53) caractéristique d'une pathologie chez un sujet mammifère (2), comprenant :

   un système (1) de génération d'un indicateur ou biomarqueur de la perception des couleurs chez un sujet mammifère conforme à la revendication 10, et
   un module de traitement (34) configuré pour déterminer un indicateur de modification (53) entre deux signaux de puissance ($SIG_{power}$, $APPROX_{power}$) générés, par le système de génération (1), à deux instants distincts ($t_1$, $t_2$) pour le même sujet en utilisant le même stimulus multicolore dynamique.

**Patentansprüche**

1. Verfahren zur Erzeugung eines Indikators oder Biomarkers ($SIG_{power}$, $APPROX_{power}$, 499) für die Farbwahrnehmung bei einem Säugetiersubjekt (2), umfassend die folgenden Schritte:

   Unterziehen (41) des Säugetiersubjekts mindestens einem dynamischen mehrfarbigen, typischerweise zweifarbigen, Stimulus, umfassend die Anzeige, auf einem Anzeigeperipheriegerät (10), eines mehrfarbigen, typischerweise zweifarbigen, Musters, von dem mindestens zwei Farben periodisch mit einer Frequenz, Markierungsfrequenz ($F_{tag}$) genannt, invertiert werden, Kontrollieren (41) einer zeitlichen Veränderung mindestens einer ($C_2$) der beiden Farben des mehrfarbigen Musters bei der Anzeige des dynamischen mehrfarbigen Stimulus, um die angezeigte Leuchtdichte dieser Farbe ändern zu lassen,
   Erfassen (42, 43, 44), mit einer Bilderfassungsvorrichtung (20), einer oszillatorischen Reaktion ($SIG_{resp}$) mindestens einer Pupille des Säugetiersubjekts bei der Anzeige des dynamischen mehrfarbigen Stimulus und
   Erzeugen (46, 48), anhand der erfassten Reaktion, eines Signals ($SIG_{power}$, $APPROX_{power}$), das für die Stärke der oszillatorischen Reaktion ($SIG_{resp}$) der Pupille in Abhängigkeit von der zeitlichen Veränderung mindestens einer der beiden Farben bei der Anzeige des dynamischen mehrfarbigen Stimulus repräsentativ ist.

2. Verfahren nach Anspruch 1, umfassend ferner einen Schritt, der darin besteht, die zweifarbige Isoleuchtdichte-Konfiguration der beiden Farben des dynamischen mehrfarbigen Stimulus zu bestimmen, die einem Minimum (MIN) des erzeugten Signals ($SIG_{power}$, $APPROX_{power}$) entspricht.

3. Verfahren nach Anspruch 1 oder 2, bei dem die andere Farbe ($C_1$) des mehrfarbigen Musters während der Anzeige des dynamischen mehrfarbigen Stimulus konstant gehalten wird.

4. Verfahren nach einem der Ansprüche 1 bis 3, bei dem die Markierungsfrequenz ($F_{tag}$) vom Subjekt (2) abhängig ist.

5. Verfahren nach Anspruch 4, umfassend ferner einen vorherigen Schritt (40) des Bestimmens der Markierungsfrequenz ($F_{tag}$), umfassend das Unterziehen des Subjekts mindestens einem Kalibrierungslichtblitz, das Messen einer mittleren Reaktionszeit der Pupille des Säugetiersubjekts auf den Kalibrierungslichtblitz und das Festlegen der Markierungsfrequenz in Abhängigkeit von der gemessenen mittleren Reaktionszeit.

6. Verfahren nach einem der Ansprüche 1 bis 5, bei dem das Säugetiersubjekt (2) zwei aufeinanderfolgenden dynamischen mehrfarbigen, typischerweise zweifarbigen, Stimuli unterzogen wird, die auf zwei unterschiedlichen Farbpaaren beruhen.

7. Verfahren nach einem der Ansprüche 1 bis 6, bei dem das erzeugte Signal ein Signal ist, das für die Oszillationsstärke der Pupille bei der Markierungsfrequenz und/oder bei einer oder mehreren ihrer Harmonischen repräsentativ ist, für den Verlauf der Frequenzkomponente, bei der Markierungsfrequenz und/oder bei einer oder mehrerer ihrer Harmonischen, der oszillatorischen Reaktion der Pupille repräsentativ ist.

8. Verfahren nach Anspruch 7, bei dem das Erzeugen (46, 48) des Signals, das für die Oszillationsstärke ($SIG_{power}$, $APPROX_{power}$) der Pupille bei der Markierungsfrequenz ($F_{tag}$) und/oder bei einer oder mehreren ihrer Harmonischen repräsentativ ist, das Anwenden (46), auf die erfasste Reaktion ($SIG_{resp}$), einer diskreten schnellen FourierTransformation über ein gleitendes Zeitfenster (Wi) und das Speichern, für jedes Zeitfenster, des Werts ($P_{i,tag}$) der Frequenzkomponente bei der Markierungsfrequenz und/oder bei einer oder mehreren ihrer Harmonischen des erhaltenen Frequenzspektrums beinhaltet.

9. Verfahren nach einem der Ansprüche 1 bis 6, bei dem das Erzeugen (46, 48) des Signals das Bestimmen einer Änderungsamplitude des Durchmessers der Pupille als Reaktion auf jede Invertierung der Farben des mehrfarbigen Musters beinhaltet, wobei das Signal ($SIG_{power}$, $APPROX_{power}$) anhand der so bestimmten Amplituden gebildet wird.

10. System (1) zur Erzeugung eines Indikators oder Biomarkers ($SIG_{power}$, $APPROX_{power}$, 499) für die Farbwahrnehmung bei einem Säugetiersubjekt (2), umfassend:

ein Anzeigeperipheriegerät (10),
ein Informationssystem (31) zur Stimulation des Säugetiersubjekts mit einem dynamischen mehrfarbigen, typischerweise zweifarbigen, Stimulus, wobei das Informationssystem die Anzeige, auf einem Anzeigeperipheriegerät, eines mehrfarbigen, typischerweise zweifarbigen, Musters steuert, von dem mindestens zwei Farben periodisch mit einer Frequenz, Markierungsfrequenz ($F_{tag}$) genannt, invertiert werden, einen Farbencontroller (32), der dazu ausgestaltet ist, mindestens eine ($C_2$) der beiden Farben des mehrfarbigen Musters bei der Anzeige des dynamischen mehrfarbigen Stimulus zeitlich zu verändern, um die angezeigte Leuchtdichte dieser Farbe ändern zu lassen,
eine Bilderfassungsvorrichtung (20), um eine oszillatorische Reaktion ($SIG_{resp}$) mindestens einer Pupille des Säugetiersubjekts bei der Anzeige des dynamischen mehrfarbigen Stimulus zu erfassen, und
einen Indikator- oder Biomarkergenerator (33), der dazu ausgestaltet ist, anhand der erfassten Reaktion ein Signal ($SIG_{power}$, $APPROX_{power}$) zu erzeugen, das für die Stärke der oszillatorischen Reaktion ($SIG_{resp}$) der Pupille in Abhängigkeit von der zeitlichen Veränderung mindestens einer der beiden Farben bei der Anzeige des dynamischen mehrfarbigen Stimulus repräsentativ ist.

11. System zur Bestimmung eines Indikators oder Biomarkers (53), der für eine Erkrankung bei einem Säugetiersubjekt (2) charakteristisch ist, umfassend:

ein System (1) zur Erzeugung eines Indikators oder Biomarkers für die Farbwahrnehmung bei einem Säugetiersubjekt nach Anspruch 10 und
ein Verarbeitungsmodul (34), das dazu ausgestaltet ist, einen Veränderungsindikator (53) zwischen zwei Stärkesignalen ($SIG_{power}$, $APPROX_{power}$) zu bestimmen, die vom Erzeugungssystem (1) zu zwei verschiedenen Zeitpunkten ($t_1$, $t_2$) für dasselbe Subjekt unter Verwendung desselben dynamischen mehrfarbigen Stimulus erzeugt wurden.

## Claims

1. Method for generating an indicator or biomarker ($SIG_{power}$, $APPROX_{power}$, 499) of the perception of colors by a mammal subject (2), comprising the following steps:

subjecting (41) the mammal subject to at least one dynamic multi-color, typically two-color, stimulus comprising displaying, on a display peripheral (10), a multi-color, typically two-color, pattern at least two colors of which are periodically inverted at a frequency called the tagging frequency ($F_{tag}$),

controlling (41) a modification over time of at least one ($C_2$) of the two colors of the multi-color pattern during the display of the dynamic multi-color stimulus, in order to make the displayed luminance of this color vary,

acquiring (42, 43, 44), with an image-acquiring device (20), an oscillatory response ($SIG_{resp}$) of at least one pupil of the mammal subject, during the display of the dynamic multi-color stimulus, and

generating (46, 48), from the acquired response, a signal ($SIG_{power}$, $APPROX_{power}$) representative of the power of the oscillatory response ($SIG_{resp}$) of the pupil as a function of the modification over time of at least one of the two colors during the display of the dynamic multi-color stimulus.

2. Method according to Claim 1, furthermore comprising a step consisting in determining the iso-luminance two-color configuration of the two colors of the dynamic multi-color stimulus corresponding to a minimum (MIN) of the generated signal ($SIG_{power}$, $APPROX_{power}$).

3. Method according to Claim 1 or 2, wherein the other color ($C_1$) of the multi-color pattern is kept fixed during the display of the dynamic multi-color stimulus.

4. Method according to one of Claims 1 to 3, wherein the tagging frequency ($F_{tag}$) is dependent on the subject (2).

5. Method according to Claim 4, furthermore comprising a prior step (40) of determining the tagging frequency ($F_{tag}$), comprising subjecting the subject to at least one calibration light flash, measuring an average response time of the pupil of the mammal subject to the calibration light flash, and setting the tagging frequency depending on the measured average response time.

6. Method according to one of Claims 1 to 5, wherein the mammal subject (2) is subjected to two successive dynamic multi-color, typically two-color, stimuli based on two different pairs of colors.

7. Method according to one of Claims 1 to 6, wherein the generated signal is a signal representative of the oscillation power of the pupil at the tagging frequency and/or at one or more of its harmonics, representative of the variation in the frequency component, at said tagging frequency and/or at one or more of its harmonics, of the oscillatory response of the pupil.

8. Method according to Claim 7, wherein the generation (46, 48) of the signal representative of the oscillation power ($SIG_{power}$, $APPROX_{power}$) of the pupil at the tagging frequency ($F_{tag}$) and/or at one or more of its harmonics comprises applying (46), to the acquired response ($SIG_{resp}$), a discrete fast Fourier transform in a moving time window (Wi) and storing in memory, for each time window, the value ($P_{i,tag}$) of the frequency component at the tagging frequency and/or at one or more of its harmonics of the obtained frequency spectrum.

9. Method according to one of Claims 1 to 6, wherein the generation (46, 48) of the signal comprises determining an amplitude of variation in the diameter of the pupil in response to each inversion of the colors of the multi-color pattern, said signal ($SIG_{power}$, $APPROX_{power}$) being formed from the amplitudes thus determined.

10. System (1) for generating an indicator or biomarker ($SIG_{power}$, $APPROX_{power}$, 499) of the perception of colors by a mammal subject (2), comprising:

   a display peripheral (10),
   a computational system (31) for stimulating the mammal subject with a dynamic multi-color, typically two-color, stimulus, the computational system controlling the display, on the display peripheral, of a multi-color, typically two-color, pattern at least two colors of which are periodically inverted at a frequency called the tagging frequency ($F_{tag}$),
   a color controller (32) configured to modify over time at least one ($C_2$) of the two colors of the multi-color pattern during the display of the dynamic multi-color stimulus, in order to make the displayed luminance of this color vary,
   an image-acquiring device (20) for acquiring an oscillatory response ($SIG_{resp}$) of at least one pupil of the mammal subject, during the display of the dynamic multi-color stimulus, and
   an indicator or biomarker generator (33) configured to generate, from the acquired response, a signal ($SIG_{power}$, $APPROX_{power}$) representative of the power of the oscillatory response ($SIG_{resp}$) of the pupil as a function of the modification over time of at least one of the two colors during the display of the dynamic multi-color stimulus.

**11.** System for determining an indicator or biomarker (53) characteristic of a pathology of a mammal subject (2), comprising:

a system (1) for generating an indicator or biomarker of the perception of colors by a mammal subject according to Claim 10, and
a processing module (34) configured to determine an indicator (53) of modification between two power signals ($SIG_{power}$, $APPROX_{power}$) generated, by the generating system (1), at two separate times ($t_1$, $t_2$) for the same subject using the same dynamic multi-color stimulus.

FIG. 1

temps

FIG. 2

FIG. 3

FIG. 4

Indicateur 1 — 50

Indicateur 2 — 51

Comparaison — 52

Indicateur de différence — 53

Seuil — 55

Comparaison — 54

Diagnostic — 56

FIG. 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2015245766 A

**Littérature non-brevet citée dans la description**

- **CAVANAGH P. et al.** *Screening for color blindness using optokinetic nystagmus,* 1984
- **CHAUDHURI A. et al.** *A new technique for estimating chromatic isoluminance in humans and monkeys,* 1990
- **PATCHE M. et al.** *Colour vision deficiencies in Alzheimer's disease,* 2003

- **PRICE M.J. et al.** *Abnormalities in color vision and contrast sensitivity in Parkinson's disease,* 1992
- **VARNEK L. et al.** *Reversible colour vision defects in obstructive jaundice,* 1981
- **ANTONETTI D.A. et al.** *Diabetic retinopathy seeing beyond glucose-induced microvascular disease,* 2006